# EUROPEAN PATENT SPECIFICATION

(11) **EP 1 702 569 B1**
(45) Date of publication and mention of the grant of the patent: **14.10.2015**
(21) Application number: 05703386.2
(22) Date of filing: 07.01.2005
(51) Int. Cl.: A61B 17/11

(54) **ANASTOMOSIS DEVICE AND METHOD OF EXCISING WALL PORTION OF IN VIVO LUMINAL ORGAN**
ANASTOMOSEVORRICHTUNG UND VERFAHREN ZUM AUSSCHNEIDEN EINES WANDTEILS EINES LUMINALEN ORGANS IN VIVO
DISPOSITIF D'ANASTOMOSE ET PROCEDE POUR EXCISER UNE PARTIE DE PAROI D'UN ORGANE LUMINAL IN VIVO

(30) Priority: 08.01.2004 JP 2004002873
(43) Date of publication of application: 20.09.2006
(73) Proprietor: Olympus Corporation, Tokyo 151-0072 (JP)
(72) Inventor: MIKKAICHI, Takayasu, 1830033 (JP)
(74) Representative: von Hellfeld, Axel
(86) International application number: PCT/JP2005/000121
(87) International publication number: WO 2005/065554

(56) References cited:
- JP-A- 2003 070 793
- US-A- 5 792 153

## Description

### TECHNICAL FIELD

The present invention relates to an anastomosis instrument and a method of excising a wall portion of a hollow organ within a living body.

Priority is claimed on Japanese Patent Application No. 2004-002873, filed January 8, 2004.

### BACKGROUND ART OF THE INVENTION

Conventionally, various anastomosis instruments and puncture needles that puncture the living body tissue have been proposed in order to inject medical solutions into the living body tissue inside a body or to perform a suturing or anastomosis operation on the living body tissue (for example, refer to PATENT DOCUMENTS 1 and 2).

PATENT DOCUMENT 3 discloses a sewing device according to the preamble of claim 1 for passing a thread through a substrate portion, particularly a portion of a patient's tissue. The device comprises a hollow needle movable between a first position in which it is out of the substrate portion and a second position in which it passes through the substrate portion. A thread carrier is attached to the thread and can be moved from a position being within the hollow needle to a position outside it. PATENT DOCUMENT 1: Japanese Unexamined Patent Application, First Publication No. 5-64642 (FIG 1)PATENT DOCUMENT 2: Published Japanese Translation No. 10-500318 of the PCT International Application (FIG. 1)). PATENT DOCUMENT 3: Published US Patent Application No. 5 792 153.

### DISCLOSURE OF THE INVENTION

### PROBLEMS TO BE SOLVED BY THE INVENTION

In conventional puncture needles and anastomosis instruments, when a plurality of the living body tissue layers are superimposed and a puncture needle is then made to protrude towards them in order to puncture only the frontmost the living body tissue layer that is closest to the puncture needle, then if the puncture needle is made to protrude more than is necessary, there is a possibility that the other the living body tissue layers that do not need to be punctured will also be punctured at the same time. Accordingly, the highest level of care and skill is required.

The present invention was conceived in view of the above described circumstances and it is an object thereof to provide an anastomosis instrument that makes it possible to accurately and easily puncture only the living body tissue that needs to be punctured, thereby resulting in it being possible to shorten the time required for an operation.

The present disclosure also provides a method of excising a wall portion of a hollow organ within a living body that, while keeping a low level of invasiveness, makes it possible to safely excise lesions such as tumors that have appeared on a wall portion of a lumen within a living body.

### MEANS FOR SOLVING THE PROBLEMS

The present invention provides an anastomosis instrument according to claim 1.

In the anastomosis instrument of the present invention, when living body tissue that is to be punctured and living body tissue that is not to be punctured are in close proximity to each other, by separating the two the living body tissues using a space retaining device, the puncture needle can be prevented from touching the living body tissue that is not to be punctured without any strict control having to be made on the protrusion length of the puncture needle. Accordingly, it is possible to accurately and easily puncture only the living body tissue that is to be punctured.

In the anastomosis instrument of the present invention, it is desirable that the anastomosis instrument further include a holding portion that holds the living body tissue that is to be punctured at a position separated from the living body tissue that is not to be punctured.

In this anastomosis instrument, by using the holding portion to hold the living body tissue that is to be punctured at a separate position from the living body tissue that is not to be punctured, the space between the two the living body tissues can be consistently maintained. Moreover, because the nearer the living body tissue that is to be punctured is held in a stationary position, the puncturing is easily performed.

It is desirable that the anastomosis instrument of the present invention further include a pinching portion that pinches the living body tissue that is to be punctured between itself and the holding portion.

In this anastomosis instrument, because the living body tissue that is to be punctured is more securely held in a stationary position by holding this the living body tissue that is to be punctured between the holding portion and the pinching portion, the puncturing can be performed even more easily.

It is desirable that the anastomosis instrument of the present invention further include a suction hole that is connected to the channel and suctions the living body tissue that is to be punctured by applying suction pressure to the interior of the channel.

In this anastomosis instrument, because the living body tissue that is to be punctured is more securely held in a stationary position by applying suction pressure to the interior of the channel and suctioning the living body tissue that is to be punctured to the suction hole, the puncturing can be performed even more easily.

In the anastomosis instrument of the present invention, it is desirable that the space retaining device be able to move between a normal position that is located in front of the distal end of the puncture needle and a withdrawal position that is separated from the front of the distal end of the puncture needle.

In this anastomosis instrument, by leaving the space retaining device in the withdrawal position and then moving it to the normal position when the two the living body tissues are to be separated, the operation prior to the separating of the two the living body tissues is made easier.

In the anastomosis instrument of the present invention, it is desirable that the space retaining device be able to be attached onto the distal end of the insertion portion.

In this anastomosis instrument, the space retaining device is fitted onto the distal end of the insertion portion and is inserted into a body cavity together with this insertion portion. As a result, the insertion of the anastomosis instrument into a body cavity is made easier.

The present disclosure provides a method of excising a wall portion of a hollow organ within a living body that includes: pulling a wall portion of a hollow organ inside a living body towards an inward side of the hollow organ; suturing full thickness of the wall portion of the hollow organ that has been pulled inwards from an inward side of the hollow organ using an anastomosis tool; and excising a portion of the wall portion of the hollow organ while the anastomosis tool is left inside the hollow organ and the wall portion of the hollow organ is left in a sutured state.

The method of excising a wall portion of a hollow organ within a living body of the present invention may be further provided with: suturing around the entire circumference of the wall portion of the hollow organ using a plurality of the anastomosis tools; and excising a portion of the wall portion of the hollow organ that has been stitched is excised around the entire circumference of the hollow organ while the plurality of anastomosis tools are left inside the hollow organ.

### ADVANTAGEOUS EFFECTS OF THE INVENTION

According to the anastomosis instrument of the present invention, it is possible to accurately and easily puncture only the living body tissue that needs to be punctured, resulting in it being possible to shorten the time required to perform an operation. According to the method of excising a wall portion of a hollow organ within a living body, it is possible to safely excise lesions such as tumors that have appeared on a wall portion of a lumen within a living body while maintaining a low level of invasiveness.

### BRIEF DESCRIPTION OF THE DRAWINGS

[FIG. 1] FIG 1 is a view showing an embodiment of the anastomosis instrument, and is a perspective view showing this anastomosis instrument and an endoscope in which this anastomosis instrument is fitted onto a distal end of an insertion portion.
[FIG. 2] FIG 2 is a cross-sectional view showing an anastomosis instrument.
[FIG 3] FIG 3 is a cross-sectional view showing principal portions of a puncturing needle that constitutes an anastomosis instrument.
[FIG 4] FIG 4 is a cross-sectional view as seen from one axial direction of a puncturing needle showing a distal end cap holding portion that constitutes an anastomosis instrument.
[FIG 5] FIG. 5 is a cross-sectional view as seen from the other axial direction of a puncturing needle showing a distal end cap holding portion that constitutes an anastomosis instrument.
[FIG 6] FIG. 6 is a phase view showing a step of an operation performed using an anastomosis instrument.
[FIG 7] FIG 7 is a phase view showing a step of an operation performed using an anastomosis instrument.
[FIG 8] FIG. 8 is a phase view showing a step of an operation performed using an anastomosis instrument.
[FIG 9] FIG 9 is a phase view showing a step of an operation performed using an anastomosis instrument.
[FIG 10] FIG 10 is a phase view showing a step of an operation performed using an anastomosis instrument.
[FIG 11] FIG. 11 is a phase view showing a step of an operation performed using an anastomosis instrument.
[FIG 12] FIG 12 is a phase view showing a step of an operation performed using an anastomosis instrument.
[FIG 13] FIG. 13 is a phase view showing a step of an operation performed using an anastomosis instrument.
[FIG 14] FIG 14 is a phase view showing a step of an operation performed using an anastomosis instrument.
[FIG 15] FIG. 15 is a phase view showing a step of an operation performed using an anastomosis instrument.
[FIG 16] FIG 16 is a phase view showing a step of an operation performed using an anastomosis instrument.
[FIG. 17] FIG. 17 is a phase view showing a step of an operation performed using an anastomosis instrument.
[FIG. 18] FIG. 18 is a phase view showing a step of an operation performed using an anastomosis instrument.
[FIG. 19] FIG. 19 is a phase view showing a step of an operation performed using an anastomosis instrument.
[FIG 20] FIG. 20 is a phase view showing a step of an operation performed using an anastomosis instrument.
[FIG. 21] FIG. 21 is a phase view showing a step of an operation performed using an anastomosis instrument.
[FIG 22] FIG 22 is a phase view showing a step of an operation performed using an anastomosis instrument.
[FIG 23] FIG. 23 is a phase view showing a step of an operation performed using an anastomosis instrument.
[FIG 24] FIG 24 is a view showing an embodiment of the anastomosis instrument of the present invention, and is a perspective view showing an anastomosis instrument that is fitted onto a distal end of an insertion portion of an endoscope.
[FIG 25] FIG 25 is a perspective view showing an anastomosis instrument that has been fitted onto a distal end of an insertion portion of an endoscope.
[FIG 26] FIG 26 is a plan view as seen from one axial direction of a puncturing needle showing an anastomosis instrument that has been fitted onto a distal end of an insertion portion of an endoscope.
[FIG 27] FIG. 27 is a phase view showing a step of an operation performed using an anastomosis instrument.
[FIG 28] FIG. 28 is a phase view showing a step of an operation performed using an anastomosis instrument.
[FIG. 29] FIG 29 is a phase view showing a step of an operation performed using an anastomosis instrument.
[FIG. 30] FIG. 30 is a phase view showing a step of an operation performed using an anastomosis instrument.
[FIG. 31] FIG. 31 is a phase view showing a step of an operation performed using an anastomosis instrument.
[FIG 32] FIG. 32 is a view showing a further embodiment of the anastomosis instrument, and is a perspective view showing this anastomosis instrument and an endoscope in which this anastomosis instrument is fitted onto a distal end of an insertion portion.
[FIG 33] FIG. 33 is a phase view showing a step of an operation performed using an anastomosis instrument.
[FIG 34] FIG 34 is a phase view showing a step of an operation performed using an anastomosis instrument.
[FIG 35] FIG 35 is a view showing a further embodiment of the anastomosis instrument, and is a sectional view showing an anastomosis instrument that is fitted onto a distal end of an insertion portion of an endoscope.
[FIG 36] FIG 36 is a phase view showing a step of an operation performed using an anastomosis instrument.
[FIG 37] FIG. 37 is a view showing a further embodiment of the anastomosis instrument, and is a perspective view showing an anastomosis instrument that is fitted onto a distal end of an endoscope.
[FIG. 38] FIG 38 is a perspective view an anastomosis instrument showing a space retaining device being moved towards the front of a puncture needle.
[FIG. 39] FIG. 39 is a view showing a further embodiment of the anastomosis instrument, and is a perspective view showing an anastomosis instrument that is fitted onto a distal end of an endoscope.
[FIG 40] FIG 40 is a view showing an embodiment of the method of excising a wall portion of a hollow organ within a living body, and is a phase view showing a step of an operation performed using an anastomosis instrument.
[FIG 41] FIG. 41 is a phase view showing a step of an operation performed using an anastomosis instrument.
[FIG. 42] FIG. 42 is a phase view showing a step of an operation performed using an anastomosis instrument.
[FIG. 43] FIG. 43 is a phase view showing a step of an operation performed using an anastomosis instrument.
[FIG 44] FIG 44 is a phase view showing a step of an operation performed using an anastomosis instrument.
[FIG 45] FIG. 45 is a phase view showing a step of an operation performed using an anastomosis instrument.
[FIG 46] FIG 46 is a phase view showing a step of an operation performed using an anastomosis instrument.
[FIG 47] FIG 47 is a phase view showing a step of an operation performed using an anastomosis instrument i.
[FIG 48] FIG 48 is a phase view showing a step of an operation performed using an anastomosis instrument.
[FIG 49] FIG 49 is a phase view showing a step of an operation performed using an anastomosis instrument.

### DESCRIPTION OF THE REFERENCE SYMBOLS

1, 35, 41, 55, 62, 81 ··· anastomosis instrument; 2 ··· endoscope; 3 ··· insertion portion; 5 ··· channel; 7 ··· puncture needle; 8 ··· nearest living body tissue; 10 ··· furthest living body tissue; 11, 37, 67, 80 ··· space retaining device; 27, 42 ··· holding portion; 28, 43, 61 ··· pinching portion; 56 ··· suction pressure source; 57 ··· suction line; 66 ··· rotation shaft.

### BEST MODE FOR CARRYING OUT THE INVENTION

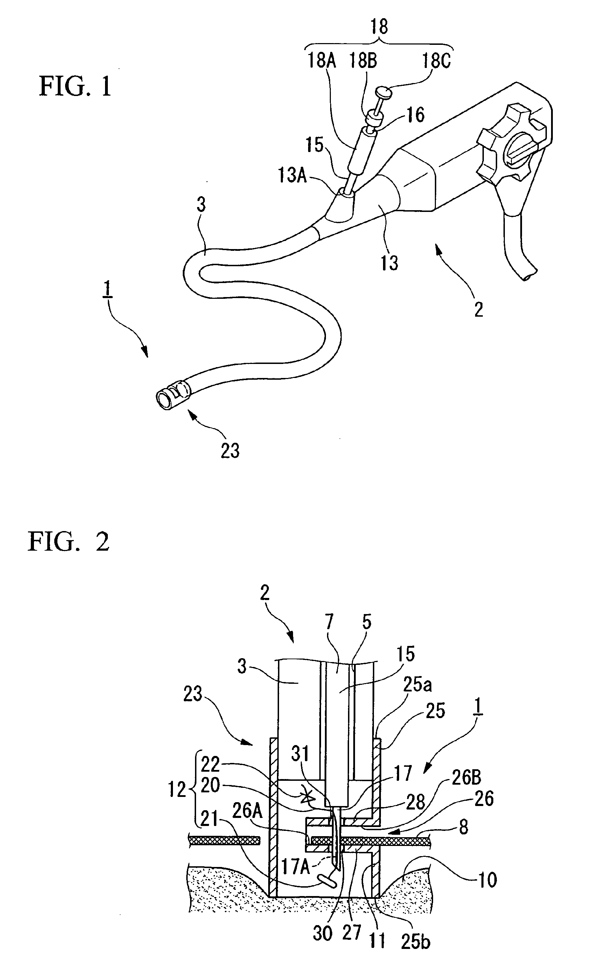

The first embodiment of the anastomosis instrument will now be described with reference made to FIGS. 1 through 23.

As is shown in FIGS. 1 through 3, an anastomosis instrument 1 of the present embodiment is provided with a puncturing needle 7, a distal end cap 23, and an anastomosis tool 12. The puncturing needle 7 moves forwards and backwards reciprocally inside a channel 5 that is provided in an insertion portion 3 of an endoscope 2 that is inserted into the interior of a body cavity, and protrudes from a distal end of the insertion portion 3 so as to puncture the living body tissue. The distal end cap 23 is fitted to a distal end of the insertion portion 3. The anastomosis tool 12 punctures the living body tissue 8 that is located in front of and nearest to a distal end of the puncture needle 7. A space retaining device 11 that holds a space between the nearest the living body tissue 8 and the furthest the living body tissue 10 to a predetermined size is provided in the distal end cap 23.

The puncture needle 7 is provided with an operating tube 15, a reciprocal movement member 16, a needle body 17, a pusher 16A, and a puncture needle operating section 18. The operating tube 15 is inserted into the channel 5 from a forceps aperture 13A that is provided in an endoscope operating section 13. The reciprocal movement member 16 is shaped like a tube and is inserted inside the operating section 15. The rod-shaped pusher 16A is inserted inside the reciprocal movement member 16. The operating tube 15, the reciprocal movement member 16, and the pusher 16A are all flexible. In addition, the operating tube 15 is able to move backwards and forwards reciprocally inside the channel 5, the reciprocal movement member 16 is able to move backwards and forwards reciprocally inside the operating tube 15, and the pusher 16A is able to move backwards and forwards reciprocally inside the reciprocal movement member 16. The needle body 17 is shaped like a tube and is connected to a distal end of the reciprocal movement member 16 so as to be inserted into a lumen. A slit 17A is formed extending in the axial direction in a tube wall of the needle body 17. A thread 20 of the anastomosis tool 12 (described below) is held in the slit 17A.

The puncture needle operating section 18 is provided with an operating section body 18A that is connected to a base end of the operating tube 15, a gripping portion 18B that is connected to a base end of the reciprocal movement member 16, and an extruding gripping portion 18C that is connected to a base end of the pusher 16A. By pushing the gripping portion 18B towards or withdrawing it away from the operating section body 18A, the puncture needle operating section 18 is able to move the reciprocal movement member 16 and the needle body 17 reciprocally inside the operating tube 15. In particular, by pushing the gripping portion 18B towards the operating section body 18A, the needle body 17 can be made to protrude from the distal end of the operating tube 15. Moreover, by pushing the extruding gripping portion 18C towards or withdrawing it away from the gripping portion 18B, the puncture needle operating section 18 is able to move the pusher 16A reciprocally inside the reciprocal movement member 16. In particular, by pushing the extruding gripping portion 18C towards the gripping portion 18B, a stopping member 21 (described below) can be pushed out from the distal end of the needle body 17.

The distal end cap 23 is formed in a cylindrical shape such that an inner diameter thereof is substantially equal to an outer diameter of the distal end of the insertion portion 3. The distal end cap 23 is fitted onto the distal end of the insertion portion 3 by inserting the insertion portion 3 into a distal end 25a of the distal end cap 23. An aperture portion 26 into which is inserted the nearest the living body tissue 8 is formed in the distal end cap 23. The aperture portion 26 is formed in an arc shape extending in the circumferential direction in a side surface of the distal end cap 23.

A holding portion 27 that holds the nearest the living body tissue 8 at a position away from the furthest the living body tissue 10, and a pinching portion 28 that pinches the nearest the living body tissue 8 between itself and the holding portion 27 are provided in the distal end cap 23. The holding portion 27 is formed on a side surface 26A that is on the distal end side of the aperture portion 26 so as to protrude towards the inner side of the distal end cap 23. The pinching portion 28 is formed on a side surface 26B that is on a base end side of the aperture portion 26 so as to protrude towards the inner side of the distal end cap 23. The holding portion 27 and the pinching portion 28 have the same shape and face each other sandwiching the aperture portion 26.

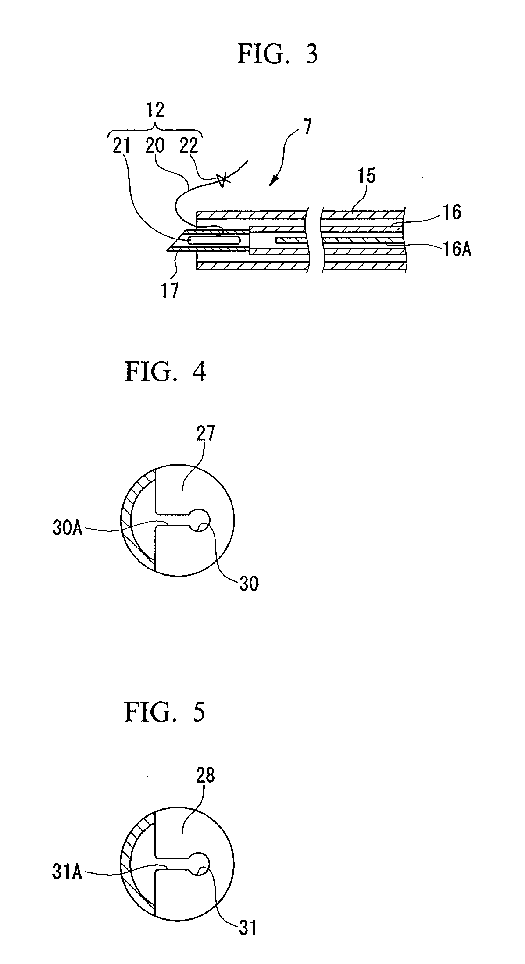

A first through hole 30 is provided in the holding portion 27 at a position that intersects an extended line of the axis of the channel 5 of the insertion portion 3 when the distal end cap 23 is fitted onto the distal end of the insertion portion 3. The first through hole 30 is formed at a size that allows only the needle body 17 of the puncture needle 7 to pass through it and prevents the operating tube 15 or the like from passing through it. Moreover, as is shown in FIG. 4, a thread extraction slit 30A for extracting via the first through hole 30 an anastomosis tool 12 that has been placed inside the needle body 17 is provided in the holding portion 27. The thread extraction slit 30A is formed extending from the first through hole 30 to an internal space inside the distal end cap 23.

A second through hole 31 is provided in the pinching portion 28 at a position that intersects an extended line of the axis of the channel 5 in the same way as in the holding portion 27. The second through hole 31 is also formed at a size that allows only the needle body 17 of the puncture needle 7 to pass through it and prevents the operating tube 15 or the like from passing through it. Moreover, as is shown in FIG. 5, a thread extraction slit 31 A for extracting via the second through hole 31 an anastomosis tool 12 that has been placed inside the needle body 17 is also provided in the pinching portion 28. The thread extraction slit 31A is formed extending from the second through hole 31 to an internal space inside the distal end cap 23.

The anastomosis tool 12 is provided with a thread 20, a stopping member 21, and a ligating tool 22. The thread 20 is positioned so as to be sandwiched in the slit 17A. The stopping member 21 is fixed to a distal end of the thread 20. The ligating tool 22 is fitted onto the thread 20 away from the stopping member 21. The ligating tool 22 can be moved when necessary along the thread 20.

A portion of the distal end cap 23 from the aperture portion 26 to a distal end 25b constitutes the space retaining device 11 that maintains the interval between the nearer the living body tissue 8 and the further the living body tissue 10 at a predetermined size. The length of the space retaining device 11 in the longitudinal direction of the distal end cap 23 is longer than the maximum length that the puncture needle 7 can protrude in order that the distal end of the needle body 17 does not reach the further the living body tissue 10 even if the puncture needle 7 is made to protrude the maximum distance from the channel 5 while the operating tube 15 is anchored by the pinching portion 28.

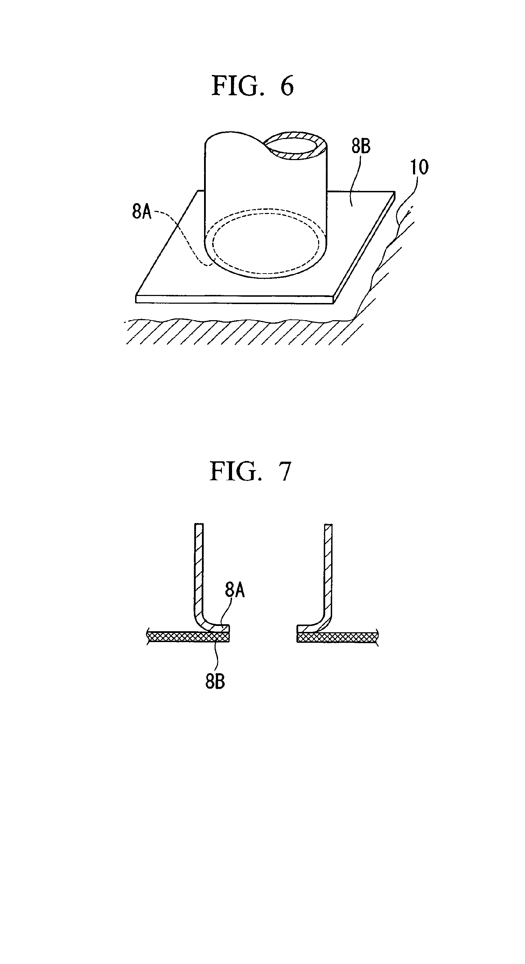

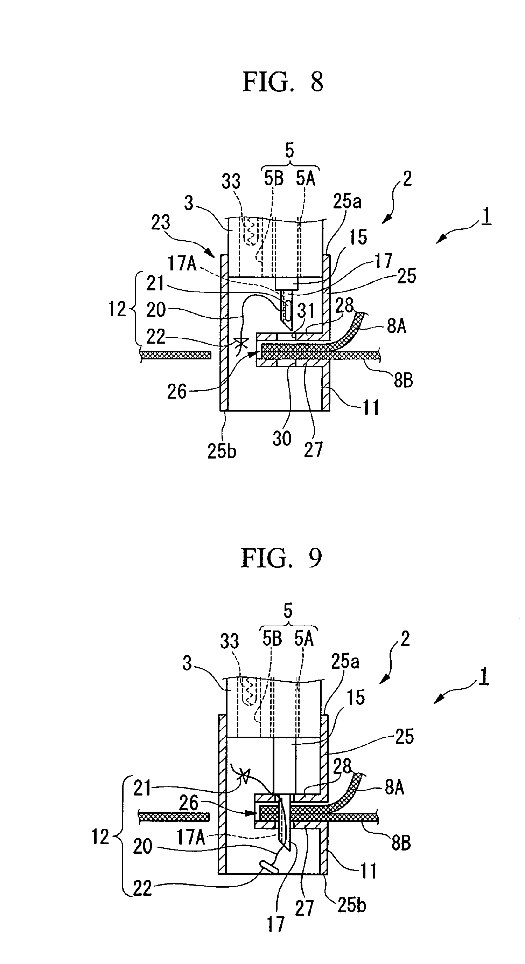

Next, a method of using the anastomosis instrument 1 of the present embodiment will be described with reference made to FIGS. 6 through 12 using as an example a case in which an anastomosis operation is performed on an end surface of a digestive tract and a wall of a different digestive organ from this. Here, as is shown in FIGS. 6 and 7, a digestive tract end surface 8A and a digestive organ wall 8B are taken as the above described nearer the living body tissue 8. Moreover, as is shown in FIGS. 8 and 9, an endoscope 2 is used in which, as the channel 5, there are provided a first channel 5A that passes through the puncture needle 7 and a second channel 5B that passes through grasping forceps 33 (described below).

Firstly, the insertion portion 3 of the endoscope 2 onto which the anastomosis instrument 1 has been fitted is inserted into a body cavity. At this time, the stopping member 21 of the anastomosis tool 12 is placed inside the needle body 17, and the thread 20 is placed in the slit 17A. Next, the puncture needle 7 is inserted in the first channel 5A, and a tube 32 and the grasping forceps 33 that have been inserted inside the tube 32 are inserted together in the second channel 5B. The tube 32 is able to move reciprocally inside the second channel 5B, and the grasping forceps 33 are able to move reciprocally inside the tube 32. Next, by rotating the insertion portion 3 and bending the distal end of the insertion portion 3 and the like, the distal end 25b of the distal end cap 23 is made to approach the digestive tract end surface 8A and the digestive organ wall 8B, and the digestive tract end surface 8A and the digestive organ wall 8B are inserted in a superimposed state into the aperture portion 26 and are placed on the holding portion 27. At this time, because the portion from the aperture portion 26 of the distal end cap 23 to the distal end 25b thereof, namely, the portion that constitutes the space holding portion 11 is placed between the digestive tract end surface 8A and digestive organ wall 8B and the further the living body tissue 10, which is a completely different organ, the digestive tract end portion 8A and digestive organ wall 8B are kept away from the further the living body tissue 10.

Next, as is shown in FIG. 8, while maintaining the distal end 25b of the distal end cap 23 in a state of contact with the further the living body tissue 10, the puncture needle 7 is made to protrude from the first channel 5A and, by performing further manipulations such that the gripping portion 18B is pushed into the operating section body 18A, the needle body 17 is made to protrude from the distal end of the operating tube 15. Next, as is shown in FIG. 9, the puncture needle 7 is moved forwards until the distal end of the operating tube 15 is placed in contact with the pinching portion 28. As a result, the needle body 17 penetrates in this order the second through hole 31, the digestive tract end surface 8A, the digestive organ wall 8B, and the first through hole 30, and thereby punctures both the digestive tract end surface 8A and the digestive organ wall 8B. As a result, the stopping member 21 is passed through the digestive tract end surface 8A and the digestive organ wall 8B. Note that, because the distal end of the operating tube 15 is anchored by the pinching portion 28, the distal end of the needle body 17 stops before it makes contact with the further the living body tissue 10.

Next, by performing further manipulations such that the extruding gripping portion 18C is pushed into the gripping portion 18B, the pusher 16A is moved forward inside the needle body 17. As a result of the pusher 16A moving forward inside the needle body 17, the stopping member 21 that has been inserted inside the needle body 17 is pushed towards the further the living body tissue 10 side. Next, by performing further manipulations such that the extruding gripping portion 18C is withdrawn from the gripping portion 18B, the pusher 16A is moved backward and is made to move inside the reciprocal movement member 16. By then manipulating the gripping portion 18B such that it is withdrawn from the operating section body 18A, the needle body 17 is retracted into the operating tube 15. Because the thread 20 is caught by the distal end of the operating tube 15 in the step to retract the needle body 17 into the operating tube 15, when the needle body 17 becomes submerged in the distal end of the operating tube 15, the thread 20 is pulled from the slit 17A, and the anastomosis tool 12 is separated from the puncture needle 7. In this manner, the thread 20 of the anastomosis tool 12 penetrates the digestive tract end surface 8A and the digestive organ wall 8B, and the stopping member 22 is placed at one end of the thread 20 while the ligating tool 22 is placed at the other end thereof with the digestive tract end surface 8A and the digestive organ wall 8B sandwiched in-between.

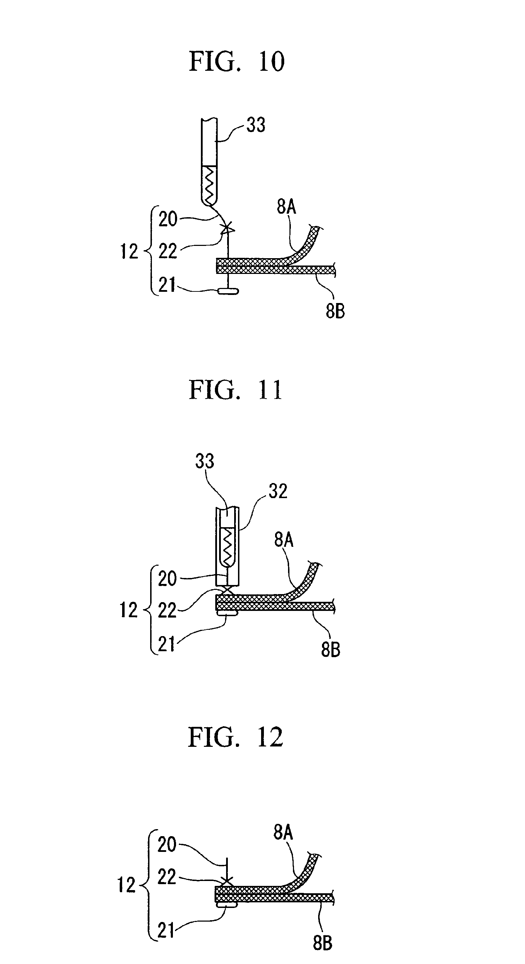

Next, as is shown in FIG 10, the grasping forceps 33 are made to protrude from the second channel 5B and grasp the base end of the thread 20. By then manipulating the grasping forceps 33 so as to pull them backwards, the stopping member 21 is pulled backwards against the digestive organ wall 8B. Next, as is shown in FIG 11, the tube 32 is withdrawn from the channel 5B and, while causing the distal end of the tube 32 to be placed against the ligating tool 22, is made to protrude from the second channel 5B. As a result, as is shown in FIG 12, the ligating tool 22 moves towards the stopping member 21 and comes up against the digestive tract end surface 8A so that the digestive tract end surface 8A and the digestive organ wall 8B are sandwiched between the stopping member 21 and the ligating tool 22. In this manner, the digestive tract end surface 8A and the digestive organ wall 8B can be placed in a state of tight contact with each other using the anastomosis tool 12.

By repeatedly performing the above described processing so that the digestive tract end surface 8A is held tightly fixed at a plurality of locations in the circumferential direction thereof, the digestive tract end surface 8A and the digestive organ wall 8B can be anastomosed.

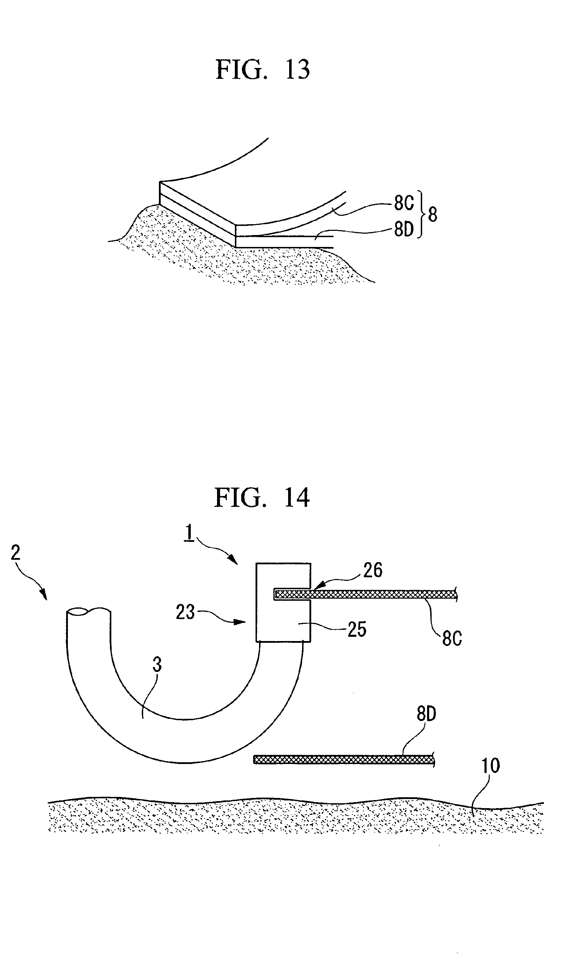

Another method of using the anastomosis instrument 1 of the present embodiment will now be described with reference made to FIGS. 13 through 17 taking as an example a case in which digestive organ walls are anastomosed together. Here, as is shown in FIG. 13, a first digestive organ wall 8C and a second digestive organ wall 8D are taken as the above described nearer the living body tissue 8.

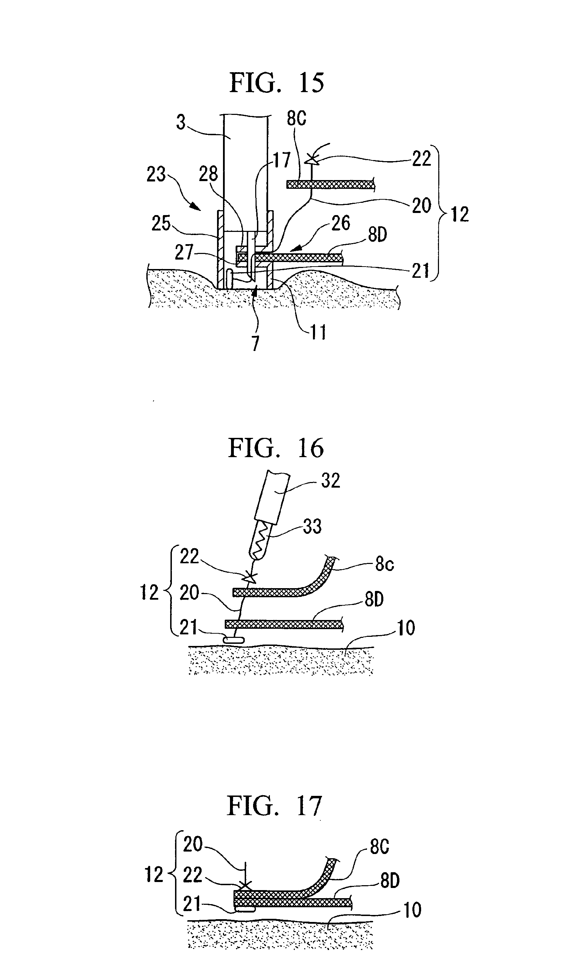

Firstly, the insertion portion 3 of the endoscope 2 to which the anastomosis instrument 1 has been fitted is inserted into a body cavity. At this time, the stopping member 21 and the ligating tool 22 of the anastomosis tool 12 are positioned in advance inside the needle body 17, and the thread 20 is placed in the slit 17A. As is shown in FIG. 14, the distal end of the insertion portion 3 to which the distal end cap 23 has been fitted is positioned between the first digestive organ wall 8C and the second digestive organ wall 8D, and is then bent away from the second digestive organ wall 8D side. Next, the first digestive organ wall 8C is inserted inside the aperture portion 26 of the distal end cap 23 and, while the distal end 25b of the distal end cap 23 is kept in a state of contact with the further the living body tissue 10, the same operations as those described above are performed so that the needle body 17 punctures the first digestive organ tract 8C. As a result, as is shown in FIG. 15, the ligating tool 22 penetrates the first digestive organ wall 8C.

Next, the second digestive organ wall 8D is inserted into the aperture portion 26 of the distal end cap 23 and, by repeating the same operations as those described above, the needle body 17 is made to protrude from the distal end of the operating tube 15 and puncture the second digestive organ wall 8D. As a result, as is also shown in FIG 15, the stopping member 21 penetrates the second digestive organ wall 8D. In this manner, the thread 20 of the anastomosis tool 12 penetrates the first digestive organ wall 8C and the second digestive organ wall 8D, and the stopping member 22 is placed at one end of the thread 20 while the ligating tool 22 is placed at the other end thereof with the first digestive organ wall 8C and the second digestive organ wall 8D sandwiched in-between.

Next, as is shown in FIG. 16, the grasping forceps 33 are made to protrude from the second channel 5B and grasp the base end of the thread 20. By then performing the same operations as those described above, as is shown in FIG 17, the ligating tool 22 moves towards the stopping member 21 and comes up against the first digestive organ wall 8C so that the first digestive organ wall 8C and the second digestive organ wall 8D are sandwiched between the stopping member 21 and the ligating tool 22. In this manner, the first digestive organ wall 8C and the second digestive organ wall 8D can be placed in a state of tight contact with each other using the anastomosis tool 12.

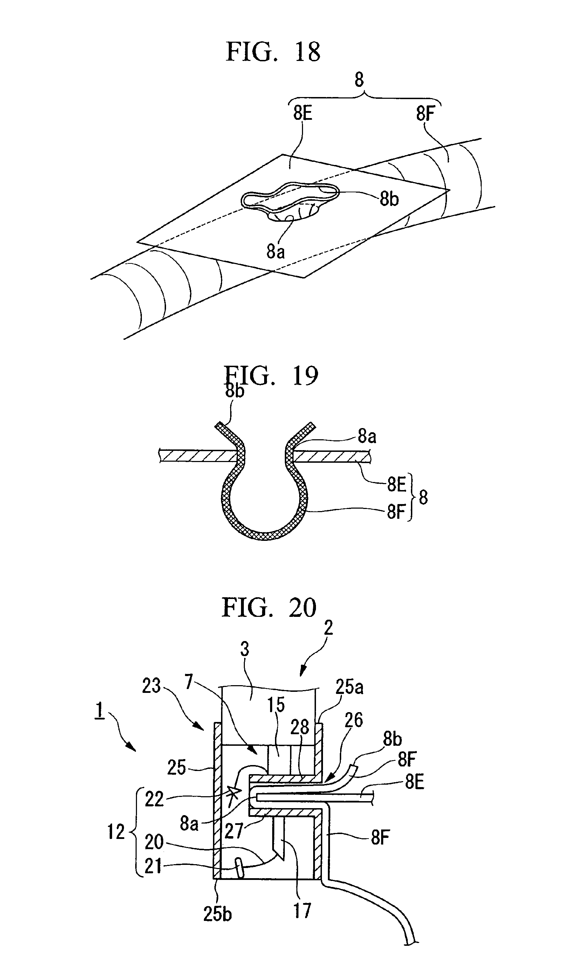

Another method of using the anastomosis instrument 1 of the present embodiment will now be described with reference made to FIGS. 18 through 22 taking as an example a case in which digestive organ walls are anastomosed together. Here, as is shown in FIGS. 18 and 19, a digestive organ wall 8E in which a first hole portion 8a is formed and a digestive tract 8F in which a second hole portion 8b is formed are taken as the above described nearer the living body tissue 8.

Firstly, the insertion portion 3 of the endoscope 2 to which the anastomosis instrument 1 has been fitted is inserted into a body cavity. At this time, the stopping member 21 of the anastomosis tool 12 is positioned in advance inside the needle body 17, and the thread 20 is placed in the slit 17A. Moreover, a circumferential edge portion of the second hole portion 8b of the digestive tract 8F is inserted in the first hole portion 8a of the digestive organ wall 8E, and the inserted circumferential edge portion of the second hole portion 8b is then folded back so as to cover a circumferential edge portion of the first hole portion 8a of the digestive organ wall 8E. In addition, the folded circumferential edge portion of the second hole portion 8b and the circumferential edge portion of the first hole portion 8a that is sandwiched by the folded circumferential edge portion of the second hole portion 8b are inserted in the aperture portion 26 of the distal end cap 23. While the distal end 25b of the distal end cap 23 is then kept in a state of contact with the nearer digestive tract 8F, the same operations as those described above are performed so that the needle body 17 punctures the digestive organ wall 8E and the digestive tract 8F. As a result, as is shown in FIG. 20, the holding member 21 penetrates the digestive organ wall 8E and the digestive tract 8F.

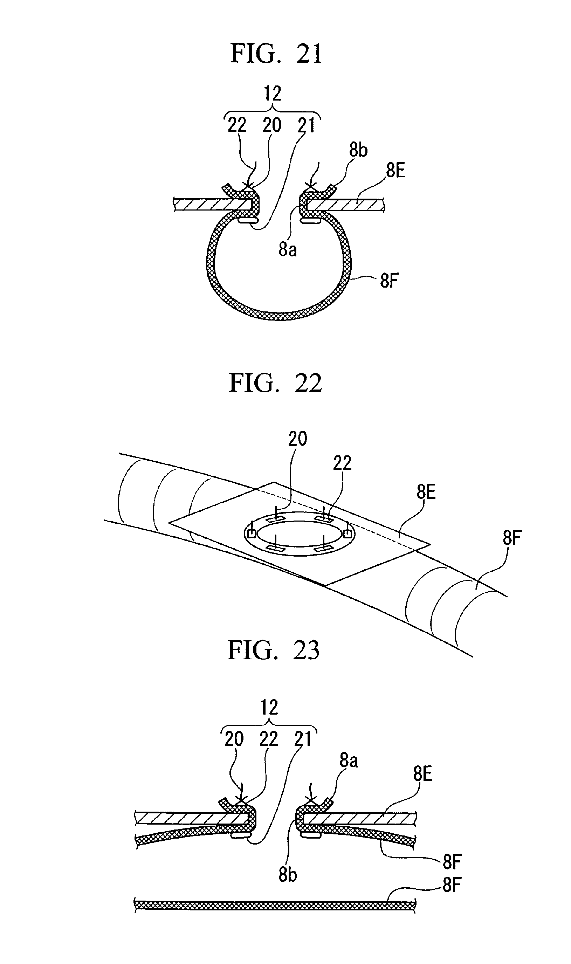

Subsequently, the same operations as those described above are repeated and, as is shown in FIG 21, the digestive organ wall 8E and the digestive tract 8F can be held in a state of tight contact with each other using the anastomosis tool 12.

By repeatedly performing the above described processing, as is shown in FIGS. 22 and 23, the digestive organ wall 8E and the digestive tract 8F are held tightly fixed at a plurality of locations in the circumferential direction of the hole portions, so that the digestive organ wall 8E and the digestive tract 8F can be anastomosed.

According to this anastomosis instrument 1, when a the living body tissue that is to be punctured, namely, the nearer the living body tissue and a the living body tissue that is not to be punctured, namely, the further the living body tissue are in close proximity to each other, by separating the two the living body tissues using the space retaining device 11, the puncture needle 7 is prevented from touching the living body tissue that is not to be punctured even without there being any strict control placed on the protrusion length of the puncture needle 7. Accordingly, it is possible to accurately and easily puncture only the living body tissue that is to be punctured.

Furthermore, according to this anastomosis instrument 1, by using the holding portion 27 to hold the nearer the living body tissue which has been separated from the further the living body tissue, the space between the two the living body tissues can be consistently maintained. Moreover, because the nearer the living body tissue which is the one to be punctured is held in a fixed position, the puncturing is easily performed.

Moreover, because the distal end cap 23 is formed in a cylindrical shape, the distal end cap 23 can be fitted easily onto the distal end of the insertion portion 3 of the endoscope 2. Furthermore, the distal end cap 23 can be easily inserted into a body cavity together with the insertion portion 3.

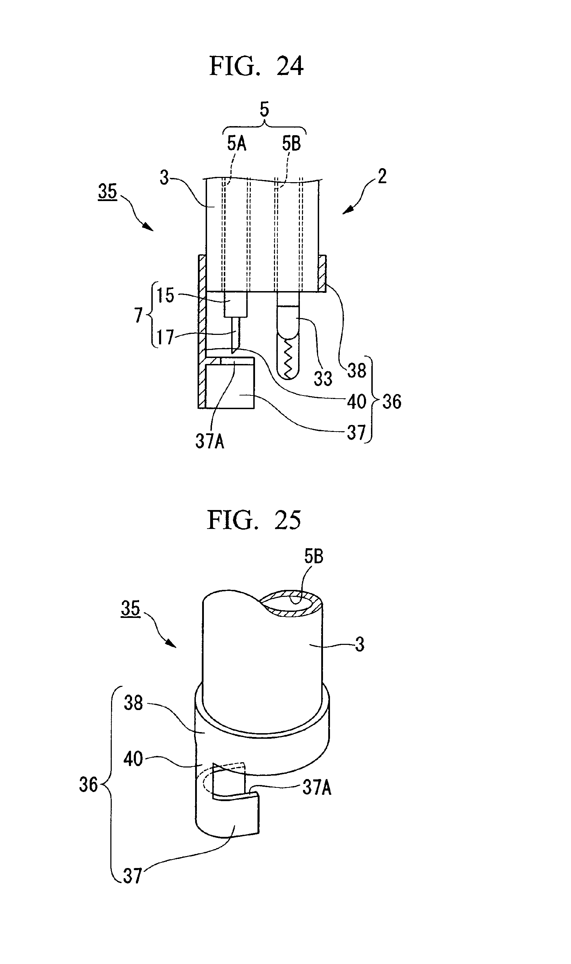

Next, an embodiment of the anastomosis instrument of the present invention will be described with reference made to FIG. 24 through FIG 31. Note that component elements that are the same as those in the above described first embodiment are given the same symbols and a description thereof is omitted.

As is shown in FIGS. 24 and 25, in an anastomosis instrument 35 of the present embodiment, a distal end cap 36 is provided with a ring-shaped fitting portion 38 whose inner diameter is substantially equal to the outer diameter of the distal end of the insertion portion 3, and a space retaining device 37 that is fixed via a connecting portion 40 to a distal end of the fitting portion 38. The distal end cap 36 is fitted onto the distal end of the insertion portion 3 by inserting the insertion portion 3 into the fitting portion 38.

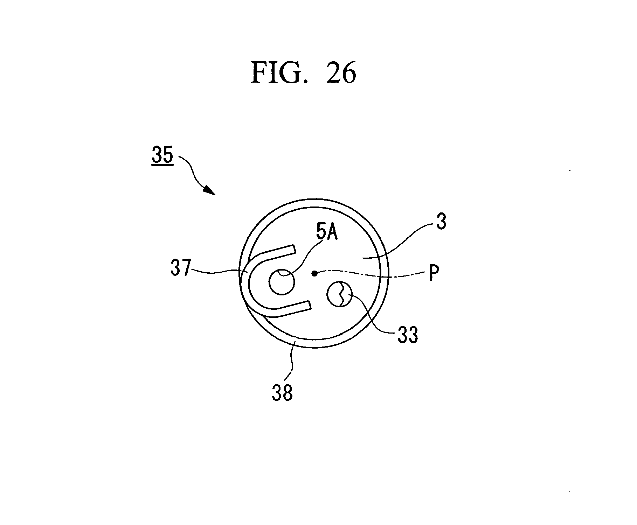

As is shown in FIG. 26, the space holding portion 37 is formed having a U-shaped cross section when viewed from the insertion direction in which the insertion portion 3 is inserted into the fitting portion 38. A U-shaped separated portion is located at a position away from the center of the fitting portion 38 and facing the center of the fitting portion 38. A side surface 37B on the fitting portion 38 side of the space retaining device 37 constitutes the holding portion. The distal end cap 36 is fitted onto the distal end of the insertion portion 3 such that, when viewed from the insertion direction, the first channel 5A is placed on the inner side of the space retaining device 37.

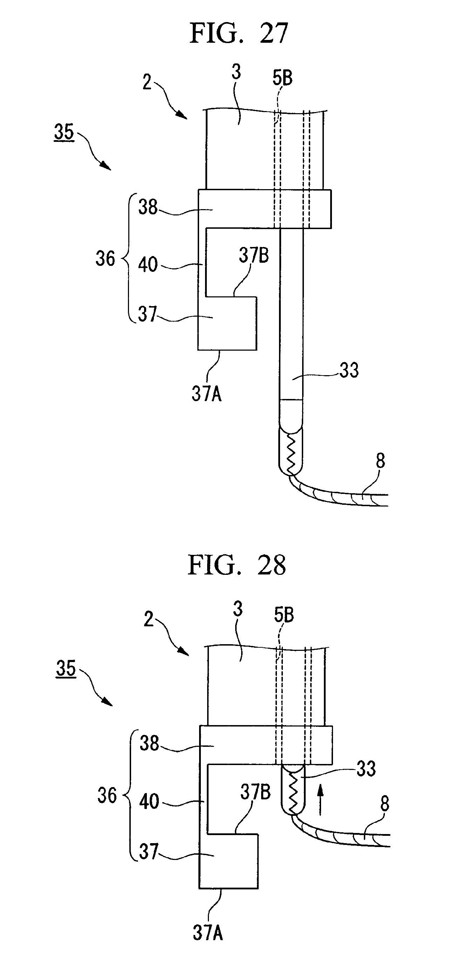

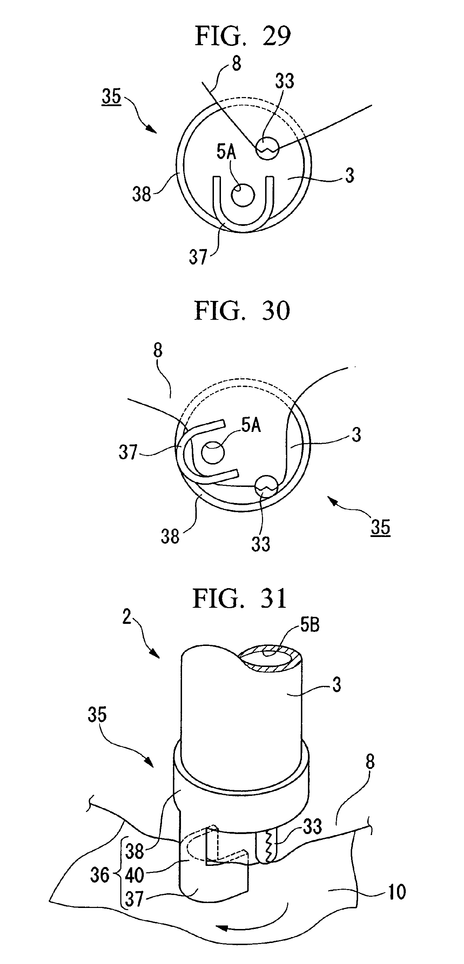

A method of using the anastomosis instrument 35 of the present embodiment will now be described with reference made to FIG 27 through 31. Firstly, the insertion portion 3 of the endoscope 2 onto which the distal end cap 36 has been fitted is inserted into a body cavity. Next, the grasping forceps 33 are made to protrude from the second channel 5B beyond a distal end side end surface 37A of the space retaining device 37 and, as is shown in FIG 27, grasp and then curl up the nearer the living body tissue 8. Next, as is shown in FIGS. 28 and 29, while maintaining their grasping on the nearer the living body tissue 8, the grasping forceps 33 are retracted into the second channel 5B. At this time, as is shown in FIGS. 30 and 31, while rotating the insertion portion 3 and bending the distal end of the insertion portion 3, the nearer the living body tissue 8 is placed on a base end side end surface 37B of the space retaining device 37. When the nearer the living body tissue 8 has been placed on the base end side end surface 37B of the space retaining device 37, the nearer the living body tissue 8 and the further the living body tissue 10 (not shown) are separated from each other. Thereafter, by performing the same operations as in the above described first embodiment, only the nearer the living body tissue 8 is punctured and anastomosed.

According to this anastomosis instrument 35, it is possible to obtain the same operational effects as those of the first embodiment. Furthermore, because the space retaining device 37 is not provided in the direction in which the grasping forceps 33 extend, when the nearer the living body tissue 8 is placed on the base end side end surface 37B of the space retaining device 37, the operation of the grasping forceps 33 is easier to conduct than in the case of the first embodiment and there is no possibility of the nearer the living body tissue 8 getting caught on the space retaining device 37.

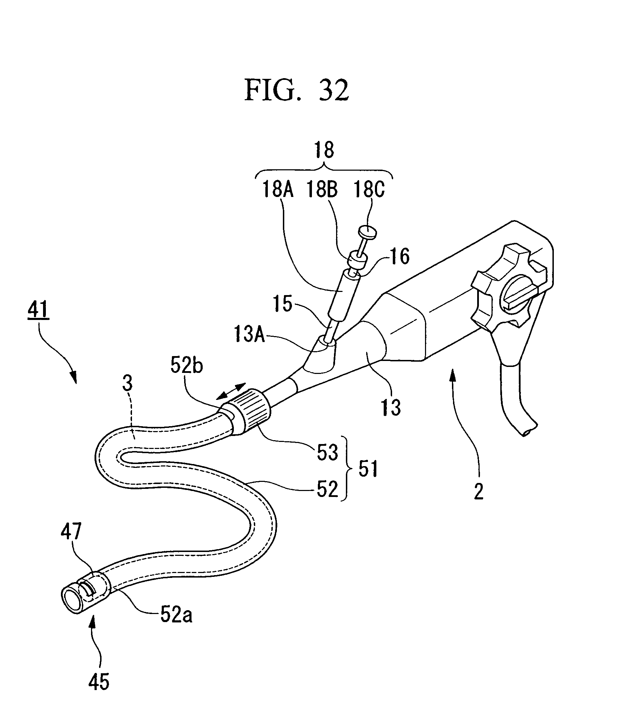

Next, a further embodiment of the anastomosis instrument will be described with reference made to FIG. 32 through FIG. 34. Note that component elements that are the same as those in the above described first embodiment are given the same symbols and a description thereof is omitted.

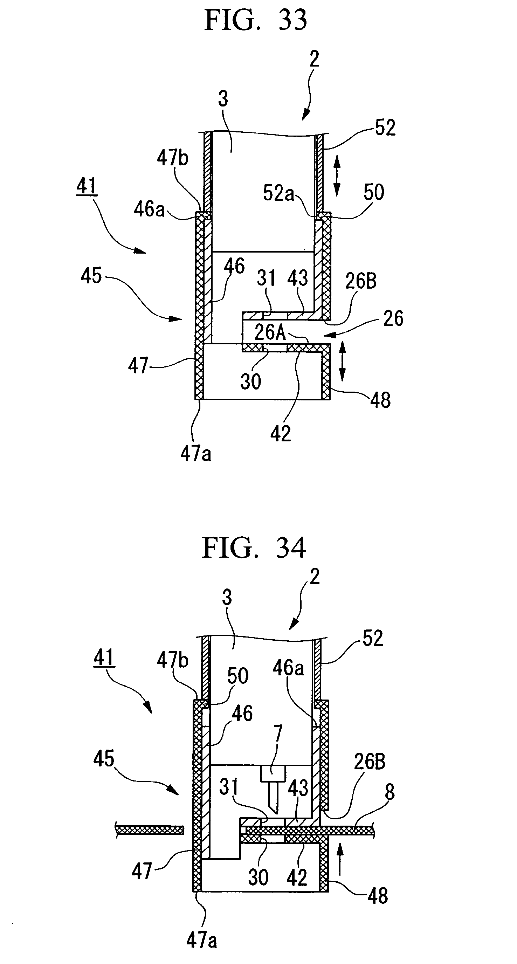

In an anastomosis instrument 41 of the present embodiment, a holding portion 42 that is provided in a distal end cap 45 is supported so as to be able to move reciprocally relative to a pinching portion 43 in the axial direction of the puncture needle. As is shown in FIG. 33, the distal end cap 45 is provided with an inner cylinder portion 46. The insertion portion 3 of the endoscope 2 is fitted into the base end portion of the inner cylinder portion 46. The distal end cap 45 is also provided with an outer cylinder portion 47 that is mounted so as to be able to slide along the exterior side of the inner cylinder portion 46. The pinching portion 43 is formed at a distal end of the inner cylinder portion 46. The aperture portion 26 and the holding portion 42 are provided on the outer cylinder portion 47. A portion extending from the distal end side end surface 26A of the aperture portion 26 to a distal end 47a of the outer cylinder portion 47 constitutes the space holding portion 48. An anchoring portion 50 that restricts the inner cylinder portion 46 such that it does not protrude from a base end 47b of the outer cylinder portion 47 is provided on the base end 47b of the outer cylinder portion 47. The anchoring portion 50 is formed in a ring shape on an inner circumferential surface of the outer cylinder portion 47. The pinching portion 43 is positioned so as to be level with the base end side end surface 26B of the aperture portion 26 when the anchoring portion 50 and a base end 46a of the inner cylinder portion 46 are placed against each other.

The anastomosis instrument 41 is provided with a tube member 51 that makes the outer cylinder portion 47 slide along the inner cylinder portion 46. As is shown in FIGS. 32 and 33, the tube member 51 is provided with a tube body 52 that has a distal end 52a that is connected to the base end 47b of the outer cylinder portion 47 and that extends as far as the endoscope operating section 13 while covering the outer circumference of the insertion portion 3. The tube member 51 is also provided with a tube operating section 53 that is connected to a base end 52b of the tube body 52 and that via the tube body 52 causes the outer cylinder portion 47 to slide along the inner cylinder portion 46.

A method of using the anastomosis instrument 41 of the present embodiment will now be described with reference made to FIGS. 33 and 34. Firstly, the insertion portion 3 of the endoscope 2 is inserted into the tube body 52 from the tube operating section 53 side and, as is shown in FIG 33, the inner cylinder portion 46 is fitted onto the distal end of the insertion portion 3. After the insertion portion 3 has been inserted into a body cavity, while rotating the insertion portion 3 and bending the distal end of the insertion portion 3, the nearer the living body tissue 8 is inserted into the aperture portion 26. By then manipulating the tube operating section 53 so that it is pulled towards the endoscope operating section 13 side, the tube body 52 is moved and the outer cylinder portion 47 is made to slide along the inner cylinder portion 46. As a result, the holding portion 42 approaches the pinching portion 43 so that the space between the two is narrowed and, as is shown in FIG. 34, the nearer the living body tissue 8 is pinched between the holding portion 42 and the pinching portion 43. Thereafter, by performing the same operations as in the above described first embodiment, only the nearer the living body tissue 8 is punctured and anastomosed.

According to this anastomosis instrument 41, by making the holding portion 42 move towards the pinching portion 43, the nearer the living body tissue 8, which is the tissue to be punctured, is sandwiched between the holding portion 42 and the pinching portion 43 and is kept in a stationary position. Accordingly, the puncturing is extremely easy.

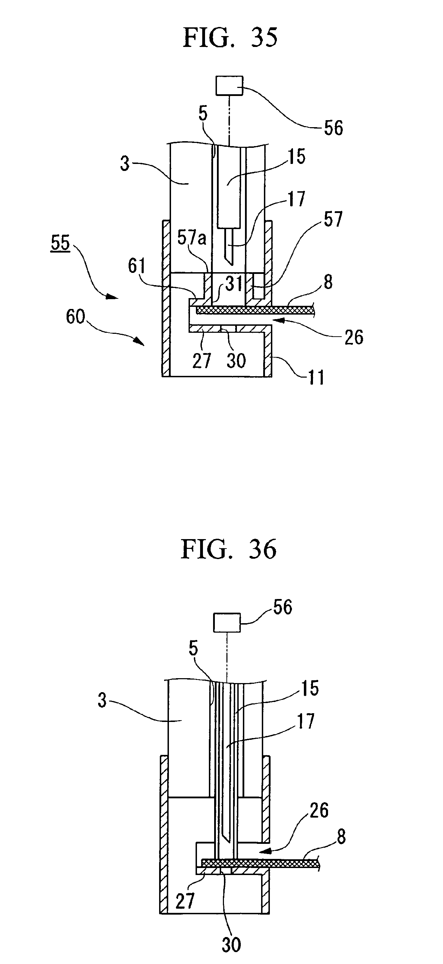

Next, a further embodiment of the anastomosis instrument will be described with reference made to FIG 35. Note that component elements that are the same as those in the above described first embodiment are given the same symbols and a description thereof is omitted.

As is shown in FIG 35, the anastomosis instrument 44 of the present embodiment is provided with an adhesion hole that holds the nearer the living body tissue 8 that is to be punctured by applying suction pressure to the interior of channel 5 of the endoscope 2 using a suction pressure source 56. The adhesion hole is formed by the second through hole 31 that is formed in a pinching portion 61. A suction line 57 that is connected to with the second through hole 31 is provided in a rear surface of the pinching portion 61. The suction line 57 protrudes to the base end side of the distal end cap 60 and is connected to the channel 5 when the distal end cap 60 is fitted onto the distal end of the insertion portion 3. The inner diameters of the second through hole 31 and the suction line 57 are formed substantially equal to the inner diameter of the channel 5.

A method of using the anastomosis instrument 55 of the present embodiment will now be given. Firstly, the distal end cap 60 is fitted onto the distal end of the insertion portion 3 and is inserted into a body cavity. While rotating the insertion portion 3 and bending the distal end of the insertion portion 3, the nearer the living body tissue 8 is then inserted into the aperture portion 26. When the suction source 56 is driven, suction pressure is applied to the interior of the channel 5 and the nearer the living body tissue 8 inside the aperture portion 26 becomes adhered to the second through hole 31. The nearer the living body tissue 8 is kept in a state of tight adhesion to the second through hole 31 for the duration of the driving of the suction source 56. Thereafter, by performing the same operations as in the above described first embodiment, only the nearer the living body tissue 8 is punctured and anastomosed.

According to this anastomosis instrument 55, by fitting the distal end cap 60 onto the distal end of the insertion portion 3, connecting together the suction line 57 and the channel 5, and then driving the suction source 56 in this state so that suction pressure is applied to the interior of the channel 5, the nearer the living body tissue 8 is adhered to the second through hole 31 and is fixed in position. As a result, the nearer the living body tissue 8 which is the one to be punctured is held in a fixed position and the puncturing is easily performed.

Note that, as is shown in FIG. 36, it is also possible for the operating tube 15 of the puncture needle 7 to be connected to the suction source 56, and for suction pressure to be applied to the interior of the operating tube 15 so that the nearer the living body tissue 8 becomes adhered to the distal end of the operating tube 15. In this case, when the suction source 56 is driven, because suction pressure is applied to the interior of the operating tube 15, the nearer the living body tissue 8 is held in a fixed position in a more stable state resulting in the puncturing becoming easier.

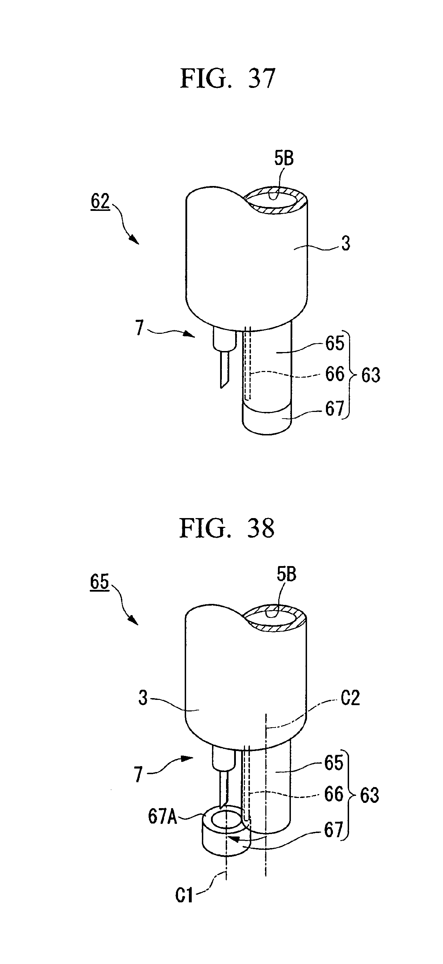

Next, a further embodiment of the anastomosis instrument will be described with reference made to FIGS. 37 and 38. Note that component elements that are the same as those in the above described first embodiment are given the same symbols and a description thereof is omitted.

In an anastomosis instrument 62 of the present embodiment, a distal end cap 63 is provided with a cap body 65 that is formed in a cylinder shape and can be removably attached to a distal end of the second channel 5B, a rotation shaft 66 that is placed off center from the center axis of the puncture needle 7 and extends in the axial direction of the cap body 65, and a space retaining device 67 that is formed in a cylinder shape and is connected to the rotation shaft 66 on the distal end side of the cap body 65 and is able to rotate around the rotation shaft 66 separately from the cap body 65.

The space retaining device 67 has substantially the same inner diameter and outer diameter as those of the cap body 65 and, depending on the angle of rotation of the rotation shaft, there are cases when a center axis C 1 of the space retaining device 67 is located on the same axis as the center axis C2 of the cap body 65 and cases when the center axis C 1 is located at a position where the needle body 17 of the puncture needle 7 can be inserted therein. Note that a base end side end surface 67A of the space retaining device 67 constitutes a supporting portion.

Next, a method of using the anastomosis instrument 62 of the present embodiment will be described. Firstly, when the center axis C 1 of the space retaining device 67 and the center axis C2 of the cap body 65 are located substantially coaxially, as is shown in FIG. 37, the distal end cap 63 is fitted into the distal end of the insertion portion 3 and is inserted into a body cavity. In addition, the rotation shaft 66 is rotated so that the space retaining device 67 is rotated around the rotation shaft 66 and, as is shown in FIG. 38, is moved to a position in front of the direction of forward movement of the puncture needle 7. In this state, the nearer the living body tissue is placed on the base end side end surface 67A of the space retaining device 67. In this manner, as is described above, the nearer the living body tissue is punctured by the puncture needle 7.

According to this anastomosis instrument 62, when the space retaining device 67 is located in a withdrawal position (i.e., the center axis C1 and the center axis C2 are positioned on the same axis) and the nearer the living body tissue is separated from the further the living body tissue, by rotating the space retaining device 67 if necessary around the rotation shaft 66 and moving it to a normal position (i.e., so that the space retaining device 67 is placed in front of the puncture needle 7), the nearer the living body tissue and the further the living body tissue can be more easily separated from each other. Furthermore, any operation prior to the separation of the two the living body tissues is more easily performed.

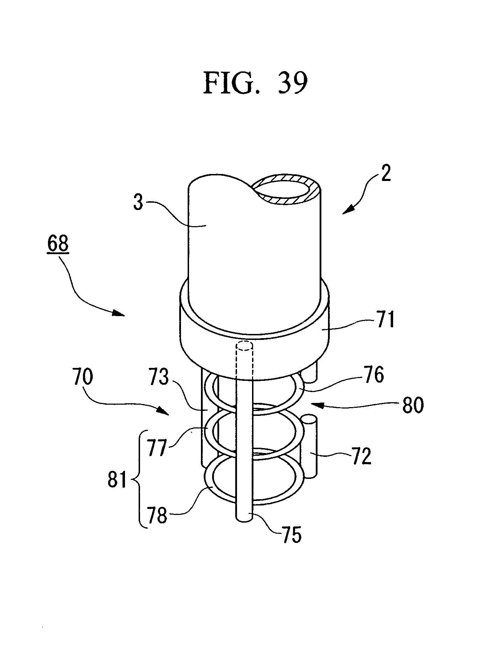

Next, a further embodiment of the anastomosis instrument will be described with reference made to FIG 39. Note that component elements that are the same as those in the above described first embodiment are given the same symbols and a description thereof is omitted.

In an anastomosis instrument 68 of the present embodiment, a distal end cap 70 is provided with a fitting portion 71 that is fitted onto the distal end of the insertion portion 3 of the endoscope 2, first through third supporting rod portions 72, 73, and 75 that have one end thereof fixed to the fitting portion 71 and have the other end thereof extending to the distal end side beyond the fitting portion 71, and first through third ring members 76, 77, and 78 that are in contact with inner sides of each of the supporting rod portions 72, 73, and 75 and are positioned having a predetermined interval between each ring in the axial direction. In the first supporting rod portion 72, the portion between the first ring member 76 and the second ring member 77 is removed so as to form an aperture portion 80. Moreover, the space retaining device 81 is formed by the second ring member 77 and the third ring member 78, and the second ring member 77 also has the function of a supporting portion.

According to this anastomosis instrument 68, when the further the living body tissue is in a state of contact against the distal end of each of the first through third supporting rod portions 72, 73, and 75, by inserting the nearer the living body tissue into the aperture portion 80 and placing it on top of the second ring member 77, it is possible to puncture only the nearer the living body tissue with the puncture needle and obtain the operational effects of each of the above described embodiments.

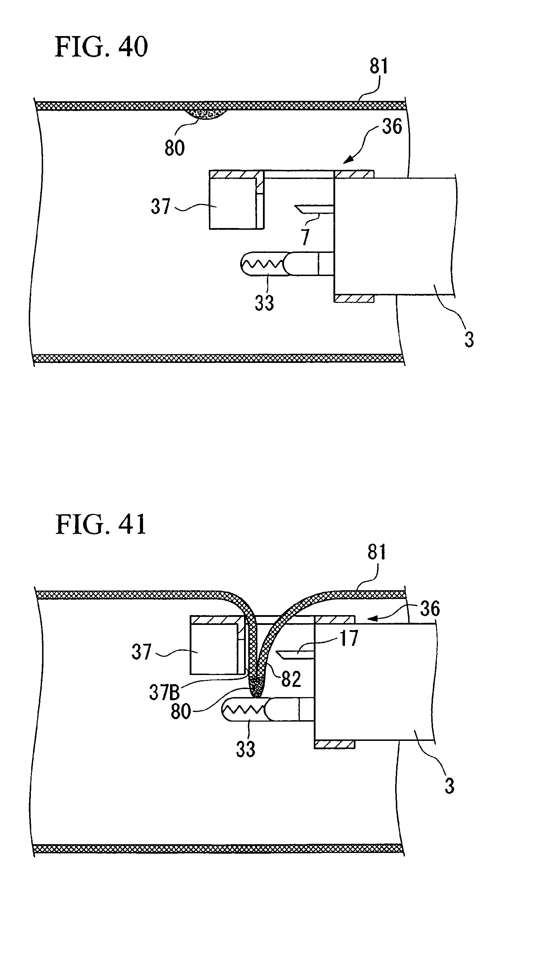

Next, an embodiment of the method of excising a wall portion of a hollow organ within a living body will be described with reference made to FIGS. 40 through 48 taking as an example a case in which a lesioned part has appeared on a hollow organ in the form of the large intestine within a living body.

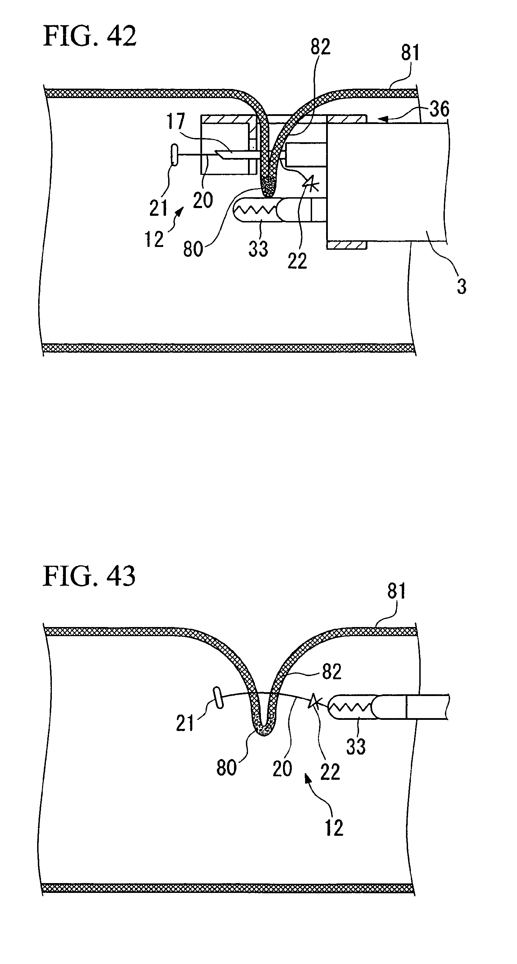

Firstly, as is shown in FIG 40, the insertion portion 3 of an endoscope that has been fitted with the anastomosis instrument 35 of the above described second embodiment is inserted into luminal tissue such as a large intestine 81 where a lesioned part 80 has appeared. The lesioned part 80 is then gripped by the grasping forceps 33 and, using the same procedure as in the above described second embodiment, is pulled towards the inner side of the lumen. As is shown in FIG. 41, a tube wall portion 82 of the large intestine 81 that includes the lesioned part 80 is then placed on the base end side end surface 37B of the space retaining device 37. Next, only the tube wall portion 82 is punctured by performing the same operation as that of the above described first embodiment. As a result, as is shown in FIG. 42, the thread 20 of the anastomosis tool 12 with the stopping member 21 fixed thereto penetrates the tube wall portion 82 that has been folded into a double layer so that the stopping member 21 becomes positioned at one end of the thread 20 and the ligating tool 22 becomes positioned at the other end of the thread 20 with the tube wall portion 82 interposed between the two.

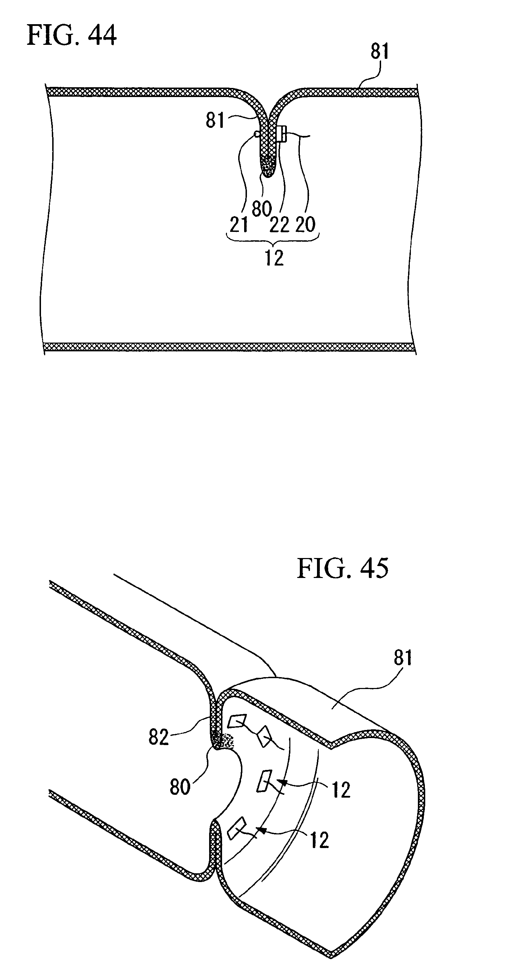

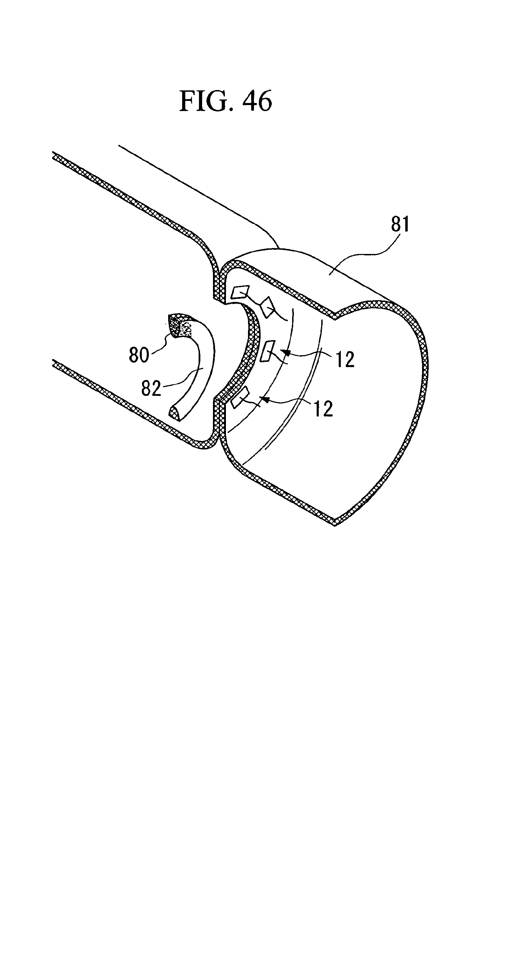

Next, as is shown in FIG 43, the grasping forceps 33 are made to protrude from the second channel 5B and grasp the base end of the thread 20. By then pulling the grasping forceps 33, the stopping member 21 is pulled back and comes up against the digestive organ wall 8B. At the same time, the ligating tool 22 is moved along the thread 20 and, as is shown in FIG 44, the double layered tube wall portion 82 becomes sandwiched between the stopping member 21 and the ligating tool 22. As a result, the tube wall portion 82 can be held in a state of being folded into a double layer. This processing is then repeated around the entire circumference of the large intestine 81 so that, as is shown in FIG. 45, the tube wall portion 82 is held firmly folded over at a plurality of locations in the circumferential direction. Next, as is shown in FIG. 46, the tube wall portion 82 that has been folded in two around the entire circumference thereof so as to protrude towards the inner side of the lumen is excised along the circumferential direction using a needle knife (not shown) leaving the portion that has been stitched together by the anastomosis instrument 12, and the lesioned part 80 is removed from the large intestine 81.

According to the above described excision method, it is possible to perform an operation using an endoscope without having to perform a laparoscopy or a normal laparotomy operation, and there is less invasiveness compared to a laparoscopy or laparotomy. Moreover, unlike a conventional automatic anastomosis instrument, the fastening force of each of the anastomosis tools 12 can be adjusted. Furthermore, by changing the stitching locations and number of the anastomosis tools 12, the present invention can deal with anastomosis diameters having a variety of sizes and in a variety of locations.

Note that, in the above described excision method, the tube wall portion 82 is excised in a circular shape around the entire circumference thereof, however, the excision does not need to be made around the entire circumference and it is also possible to excise only the portion in the circumferential direction that includes the lesioned part 80.

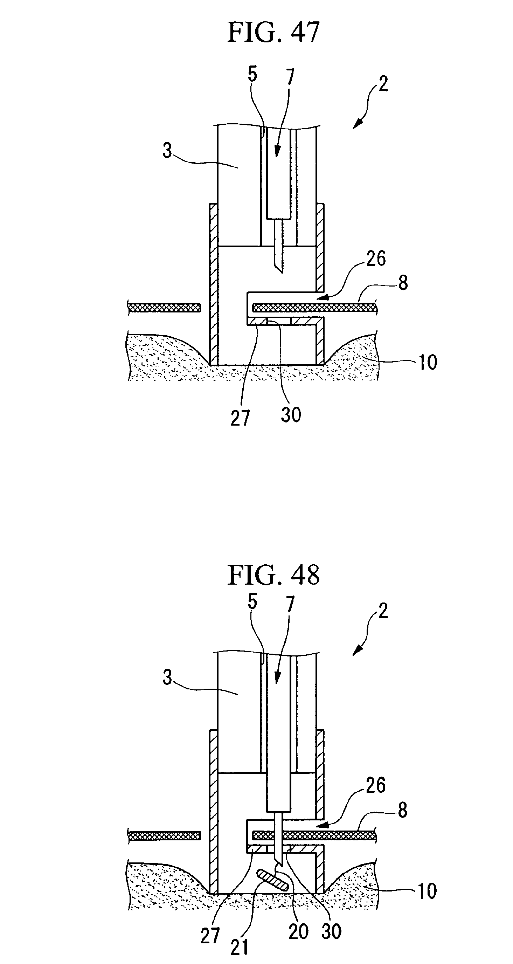

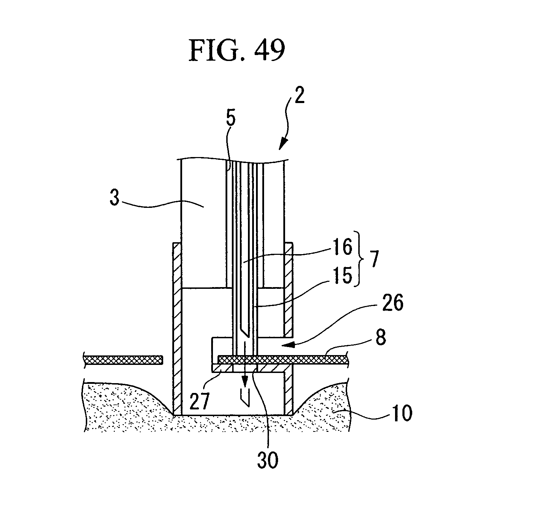

While preferred embodiments of the invention have been described and illustrated above, it should be understood that these are exemplary of the invention and are not to be considered as limiting. For example, the luminal tissue is not limited to the large intestine and the present invention may also be used for the small intestine, the stomach, the duodenum, and the esophagus and the like. As is shown in FIGS. 47 and 48, it is still possible to puncture the nearer the living body tissue 8 that has been inserted into the aperture portion 26 even if a portion corresponding to the pinching portion is not provided. Moreover, as is shown in FIG 49, it is also possible to cause the operating tube 15 of the puncture needle 7 to protrude and push against the holding portion 27 so that the nearer the living body tissue 8 is pinched between the operating tube 156 and the holding portion 27. In this case as well, it is possible to puncture only the nearer the living body tissue 8 with the nearer the living body tissue 8 and the further the living body tissue 10 in a state of separation from each other.

### INDUSTRIAL APPLICABILITY

The present invention relates to an anastomosis instrument that includes a puncture needle that moves reciprocally inside a channel that is provided in an insertion portion that is inserted into a body cavity, and that protrudes from a distal end of the insertion portion so as to puncture the living body tissue; and a space retaining device that retains at a fixed size a space between the living body tissue that is to be punctured that is loaded in the distal end of the insertion portion and is positioned in front of a distal end of the puncture needle, and the living body tissue that is not to be punctured that is positioned further from the puncture needle than is the living body tissue to be punctured. According to the anastomosis instrument of the present invention, it is possible to more accurately and easily puncture only the living body tissue that needs to be punctured, resulting in it being possible to shorten the time required to perform an operation.

## Claims

1. An anastomosis instrument (35) adapted to be used with an endoscope (2), the anastomosis instrument (35) comprising:
a puncture needle (7) adapted to be moved reciprocally inside a first channel (5A) that is provided in an insertion portion (3) of the endoscope (2) that the insertion portion (3) has the first channel (5A) and a second channel (5B), the insertion portion (3) is adapted to be inserted into a body cavity, and the puncture needle (7) is adapted to protrude from a distal end of the insertion portion (3) so as to puncture a living body tissue (8); and
a space retaining device (37) that is positioned in front of a distal end of the puncture needle (7), and that provides a predetermined clearance between the living body tissue (8) that is to be punctured that is loaded in the distal end of the insertion portion (3), and the living body tissue (10) that is not to be punctured that is positioned further from the puncture needle (7) than the living body tissue (8) that is to be punctured;
**characterized in that** the anastomosis instrument (35) further comprises:
a distal end cap (36) having a ring shaped fitting portion (38) adapted to be fitted onto a distal end of the insertion portion (3); and
a connecting portion (40) which fixes the space retaining device (37) and the distal end of the fitting portion (38); wherein
an inner diameter of the fitting portion (38) is substantially equal to an outer diameter of the distal end of the insertion portion (3), and
the space retaining device (37) is located at a position away from the center of the fitting portion (38) when viewed from an insertion direction in which the insertion portion (3) is inserted into the fitting portion (38) so that the space retaining device is not provided in the direction in which a grasping forceps (33) protrudes from the second channel (5B).

2. The anastomosis instrument (35) according to claim 1, wherein
when the fitting portion (38) is positioned with the insertion portion (3), and when viewed along the insertion direction, the space retaining device (37) has a U-shaped cross section and the first channel (5A) is placed on the inner side of the space retaining device (37).

## Patentansprüche

1. Anastomosegerät (35), das dazu eingerichtet ist, mit einem Endoskop (2) benutzt zu werden, wobei das Anastomosegerät (35) umfasst:
eine Punktionsnadel (7), die dazu eingerichtet ist, in einem ersten Kanal (5A) hin- und herbewegt zu werden, der in einem Einführabschnitt (3) des Endoskops (2) vorgesehen ist, wobei der Einführabschnitt (3) den ersten Kanal (5A) und einen zweiten Kanal (5B) aufweist, der Einführabschnitt (3) dazu eingerichtet ist, in einen Körperhohlraum eingeführt zu werden und die Punktionsnadel (7) dazu eingerichtet ist, von einem distalen Ende des Einführabschnitts (3) vorzustehen, um ein lebendes Körpergewebe (8) zu punktieren; und
eine Raumerhaltungseinrichtung (37), die vor einem distalen Ende der Punktionsnadel (7) angeordnet ist und die einen vorbestimmten Freiraum zwischen dem zu punktierenden lebenden Körpergewebe (8), das in das distale Ende des Einführabschnitts (3) geladen ist, und dem nicht zu punktierenden lebenden Körpergewebe (10) bereitstellt, das weiter entfernt von der Punktionsnadel (7) ist als das zu punktierende lebende Körpergewebe (8);
**dadurch gekennzeichnet, dass** das Anastomosegerät (35) ferner umfasst:
eine distale Endkappe (36) mit einem ringförmigen Passabschnitt (38), der dazu eingerichtet ist, auf ein distales Ende des Einführabschnitts (3) aufgesteckt zu werden; und
einen Verbindungsabschnitt (40), der die Raumerhaltungseinrichtung (37) und das distale Ende des Passabschnitts (38) fixiert; wobei
ein Innendurchmesser des Passabschnitts (38) im Wesentlichen gleich einem Außendurchmesser des distalen Endes des Einführabschnitts (3) ist, und
die Raumerhaltungseinrichtung (37), betrachtet aus einer Einführrichtung, in der der Einführabschnitt (3) in den Passabschnitt (38) eingeführt ist, an einer von dem Zentrum des Passabschnitts (38) entfernten Position angeordnet ist, so dass die Raumerhaltungseinrichtung nicht in der Richtung vorgesehen ist, in die eine Greifzange (33) von dem zweiten Kanal (5B) vorsteht.

2. Anastomosegerät (35) gemäß Anspruch 1, bei dem,
wenn der Passabschnitt (38) mit dem Einführabschnitt (3) positioniert ist, die Raumerhaltungseinrichtung (37), betrachtet entlang der Einführrichtung, einen U-förmigen Querschnitt hat und der erste Kanal (5A) auf der Innenseite der Raumerhaltungseinrichtung (37) platziert ist.

## Revendications

1. Instrument d'anastomose (35) adapté à être utilisé avec un endoscope (2), l'instrument d'anastomose (35) comprenant :
une aiguille de perforation (7) adaptée à être déplacée en va et vient à l'intérieur d'un premier canal (5A) qui est prévu dans une partie d'insertion (3) de l'endoscope (2), la partie d'insertion (3) a le premier canal (5A) et un deuxième canal (5B), la partie d'insertion (3) est adaptée à être insérée dans une cavité corporelle, et l'aiguille de perforation (7) est adaptée à faire saillie à partir d'une extrémité distale de la partie d'insertion (3) de façon à perforer un tissu (8) d'un corps vivant ; et
un dispositif de maintien d'espace (37) qui est positionné en avant d'une extrémité distale de l'aiguille de perforation (7), et qui prévoit un espace prédéterminé entre le tissu (8) du corps vivant qui doit être perforé qui est chargé dans l'extrémité distale de la partie d'insertion (3), et le tissu (10) du corps vivant qui ne doit pas être perforé qui est positionné plus loin de l'aiguille de perforation (7) que le tissu (8) du corps vivant qui doit être perforé ;
**caractérisé en ce que** l'instrument d'anastomose (35) comprend en outre :
une coiffe (36) d'extrémité distale ayant une partie d'ajustement (38) en forme d'anneau adaptée à être ajustée sur une extrémité distale de la partie d'insertion (3) ; et
une partie de connexion (40) qui fixe le dispositif de maintien d'espace (37) et l'extrémité distale de la partie d'ajustement (38) ; dans lequel
un diamètre intérieur de la partie d'ajustement (38) est sensiblement égal à un diamètre extérieur de l'extrémité distale de la partie d'insertion (3), et
le dispositif de maintien d'espace (37) est situé en une position à l'écart du centre de la partie d'ajustement (38), vu d'un sens d'insertion dans lequel la partie d'insertion (3) est insérée dans la partie d'ajustement (38) de telle sorte que le dispositif de maintien d'espace n'est pas prévu dans le sens dans lequel une pince de préhension (33) fait saillie du deuxième canal (5B).

2. Instrument d'anastomose (35) selon la revendication 1, dans lequel
lorsque la partie d'ajustement (38) est positionnée avec la partie d'insertion (3), et lorsque vu le long du sens d'insertion, le dispositif de maintien d'espace (37) a une section transversale en forme de U et le premier canal (5A) est placé sur le côté intérieur du dispositif de maintien d'espace (37).
